# EUROPEAN PATENT APPLICATION

(11) **EP 0 806 183 A1**
(43) Date of publication of application: **12.11.1997**
(21) Application number: 97201384.1
(22) Date of filing: 09.05.1997
(51) Int. Cl.: A61B 17/32

(54) **Tissue morcellator**

(30) Priority: 10.05.1996 BE 9600421
(71) Applicant: SATURNUS A.G., L-2960 Luxembourg (LU)
(72) Inventor: The designation of the inventor has not yet been filed
(74) Representative: Prins, Hendrik Willem

(57) **Abstract**

The invention relates to a tissue morcellator comprising a tube provided at one end with rotatable cutting means which are left at least partly free radially relative to the tube, wherein the cutting means preferably lie at an angle of 10-90°, preferably 20-80°, relative to the tube axis.

## Description

The present invention relates to a tissue morcellator, in particular to an endoscopic tissue morcellator which can be used for cutting off and removing tissue.

The known tissue morcellators take for instance the form of a straight hollow cylindrical tube, the free end of which is provided with a toothing, so that by rotating this tube a cylinder of tissue can be bored out. According to a more elaborate form of this morcellator, the cylinder which is provided at its end with a toothing for cutting off tissue is received in an outer tube which is stationary while the inner tube is driven, again in order to remove a cylindrical piece of tissue.

These known morcellators have the drawback that the cutting means act only in axial direction, whereby only cylinders of tissue are bored out. This boring has the drawback that it cannot be determined how far it is possible to bore, whereby there is the danger that underlying tissue which must not be removed is damaged, blood vessels can be contacted and that much boring is necessary to remove a relatively large organ such as a uterus. A further drawback is that before tissue can be removed with this morcellator the relevant organ must be exposed as much as possible. A final drawback is that these morcellators cannot be used at the beginning of the surgical operation to reduce the volume of an organ for removing.

The present invention has for its object to modify these known morcellators in the sense that it is now possible to remove tissue with this morcellator by moving the morcellator in a direction oblique to the axial direction or perpendicular thereto, i.e. the radial direction.

This is achieved according to the invention with a tissue morcellator comprising a tube provided at one end with rotatable cutting means which are left at least partly free radially relative to the tube. By directing the tube laterally and optionally slightly axially it is thus possible to continuously remove strips of tissue without the tissue herein being cut to greater depth. The tissue is thus removed by performing solely zigzag movements over a slightly increasing depth. The above described drawbacks are thus substantially avoided.

Depending on the surgical operation to be used and depending on the organ for removal, it is further recommended that the cutting means lie at an angle of 10-90°, preferably 20-80°, relative to the tube axis. It is thus possible to choose the cutting depth. A more universally applicable tissue morcellator is obtained if in further preference the angle is adjustable relative to the tube axis.

In order to be able to give the tissue morcellator a rigid form and enable easy sterilization and convenient use thereof, it is further recommended that the cutting means and the drive means connected thereto are mounted in an inner tube received in the morcellator tube.

If the inner tube is substantially clear it is possible for the severed tissue which can be cut out in the shape of a curved cylinder to be discharged as such a cylinder, whereby later microscopic examinations are very well possible.

The discharge of tissue can be further improved if in further preference discharge means are arranged in the inner tube for discharging severed tissue. Examples of such discharge means comprise a so-called Archimedean screw and flushing means.

According to another embodiment the cutting means are mounted with bearing means on an outer tube and drivable with an inner tube received in the outer tube. A morcellator is thus provided whereof the inner tube is substantially wholly free and, if an annular knife is used, a maximum entrance opening is also present. If in further preference the bearing means of the cutting means are connected to a fastening ring mounted releasably on the outer tube, it is possible to exchange the cutting means quickly and simply, which is advantageous in the case the cutting means and also the remaining parts of the morcellator must be separately sterilizable. It is moreover possible to apply, if desired and depending on the procedure, different types of cutting means in an easily exchangeable manner in the same morcellator.

If in further preference the bearing means are connected via hinge means and via a hinge member to the outer tube it is possible to adjust as required the cutting position of the cutting means relative to the morcellator axis. This adjustability can be realized in simple and adequate manner if more preferably the hinge member is a member provided with heating means and containing memory metal.

It will be apparent that the invention is certainly not limited to the discharge of tissue strips which have been removed by lateral movement of the morcellator through the tissue, but that cutting means can also be applied with which the tissue is already diminuted to a greater or lesser extent during severing.

Mentioned and other features of the tissue morcellator according to the invention will be further elucidated hereinbelow with reference to a number of embodiments which are only given by way of example without the invention being deemed limited thereto.

In the drawing:
figure 1 shows a schematic view of an endoscopic surgical operation for removing at least a part of the uterus using a morcellator according to the invention;
figure 2 shows a partly broken away view of detail II of figure 1;
figure 3 shows schematically on larger scale a variant of the cutting means as designated with detail III in figure 2;
figure 4 shows another variant;
figure 5 is a schematic view corresponding with figure 1 of another morcellator according to the invention;
figure 6 shows a variant of detail XI in figure 5;
figure 7 shows another variant corresponding with figure 6;
figure 8 is a view from another angle of view of the morcellator shown in figure 7; and
figures 9 and 10 each show yet another variant.

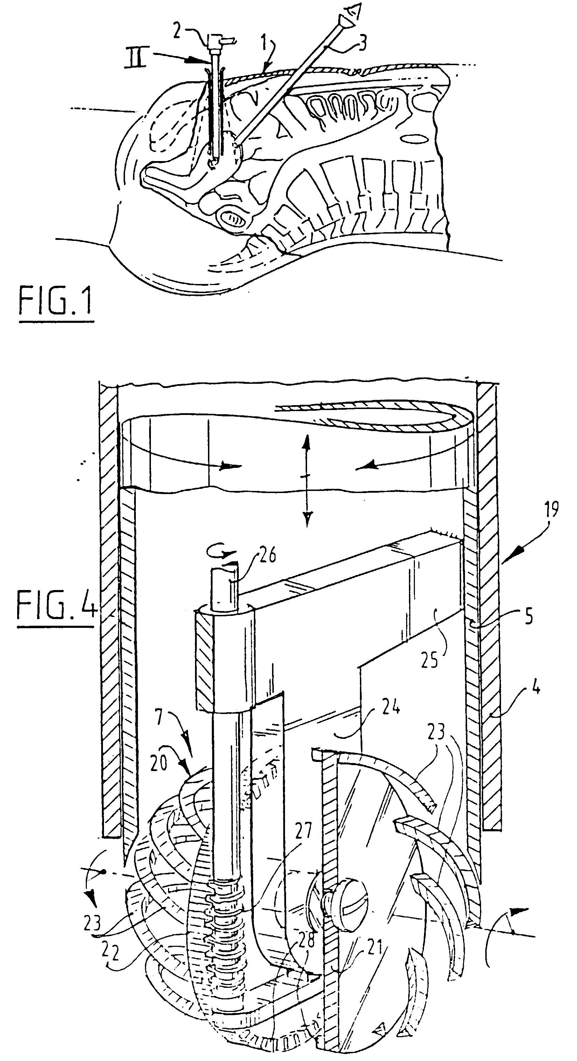

Figure 1 shows an endoscopic surgical operation, wherein in an abdominal cavity 1 at least a part of the uterus is removed with a morcellator according to the invention, wherein the operation is observed via an endoscope 3.

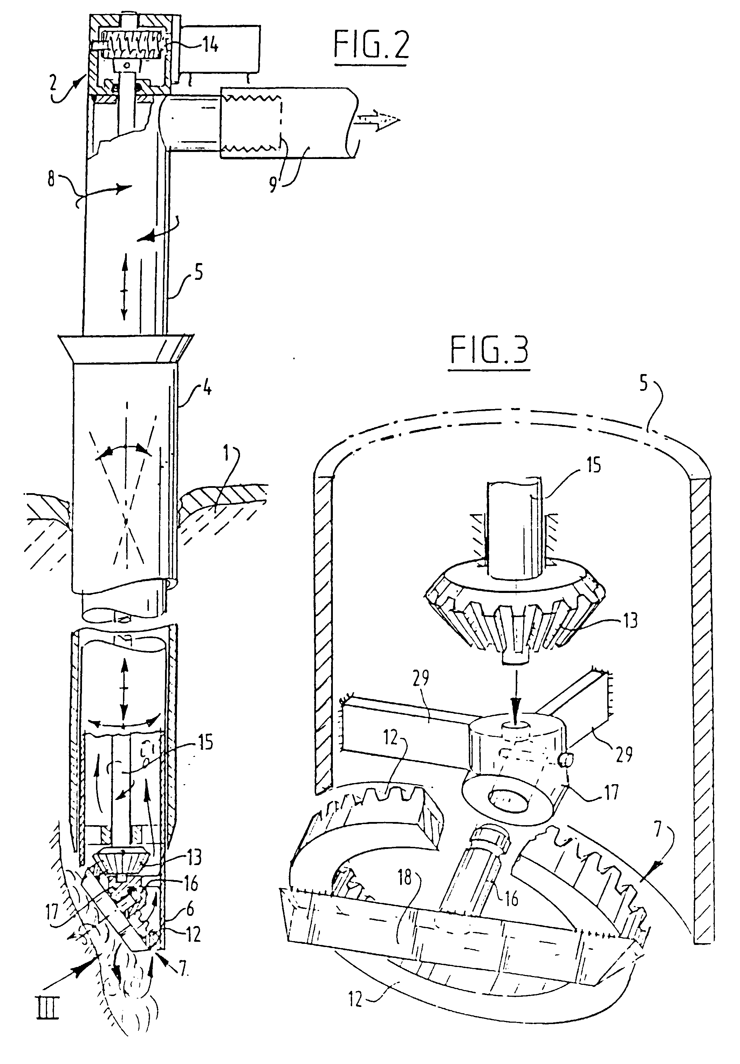

Figure 2 shows in more detail the tissue morcellator 2 according to the invention. This comprises a tube 4 which is inserted into the abdominal cavity and an inner tube which is received in this tube 4 and which is received axially slidably and rotatably in tube 4. The lower end 6 of inner tube 5 is provided with cutting means 7 which at this lower end 6 lie radially free relative to tubes 4 and 5. Tissue 10 which is severed with cutting means 7 is discharged via inner tube 5 via the outlet 9.

The cutting means 7 comprise an annular knife 11 which is provided on its rear end and side with a gear rim 12 which is in engagement with a bevel gear 13 which is driven via shaft 15 using drive means 14. The annular knife 11 is provided with a shaft end part 16 which is received for rotation and in releasably snapping manner in a support piece 17 (which will be discussed in further detail in figure 3). It will be apparent that if shaft 15 is mounted close to the wall of inner tube 5 the latter is left substantially clear and a strip of severed tissue 10 can be discharged as a whole via the tube and the outlet 9. If use is made of a flexible shaft, traction can further be applied to tissue for separating via the tube using a clamp, whereby this tissue can be removed easily.

Another variant is shown in figure 3 wherein cutting means 7 now take the form of a knife blade 18 which is mounted on the gear rim 12. It can be seen clearly that support piece 17 is fixed to inner tube 5 using wings 29. Using the morcellator as shown in figure 3 the tissue will be broken up into smaller pieces and can be transported upward by flushing or by including a known Archimedean screw in inner tube 5.

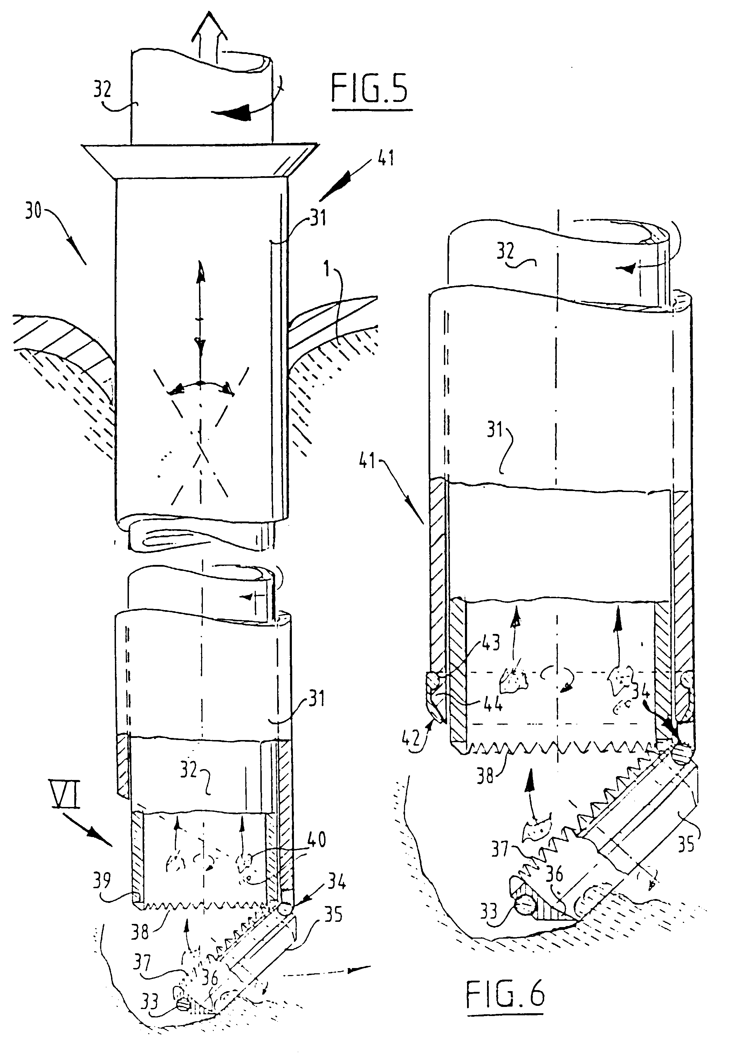

Figure 4 shows another morcellator 19 according to the invention wherein at the lower end of tubes 4 and 5 the cutting means substantially take the form of a cutting ball 20 which is constructed from two discs 21, 22 provided with knife arcs 23. Discs 21, 22 are mounted on a bearing piece 24 which is connected via a wing 25 to inner tube 5. Discs 21, 22 are driven with a drive shaft 26 which ends in a worm 27 which is in driving contact with the toothings 28 of the two discs 21, 22.

It will be apparent that with the morcellator 19 according to the invention it is possible to cut in all radial directions since these cutting means 7 are now left radially clear on all sides relative to tubes 4 and 5.

Figure 5 shows a morcellator 30 according to the invention comprising a morcellator outer tube 31 and a rotatable inner tube 32 received co-axially therein. Outer tube 31 is provided on its underside with a mounting means 34 embodied as a ring 33 in which an annular knife 35 is mounted for rotation which is provided on one end with an annular cutting edge 36 and on its other side with a toothing 37. This toothing 37 combs with a toothing 38 on the free end 39 of inner tube 32. The knife 35 is thus mounted on and drivable with the outer tube which is otherwise left completely clear for discharge of tissue 40 severed with knife 35.

Figure 6 shows a morcellator 41 according to the invention which substantially corresponds with the morcellator of figure 5. In this case the bearing ring 33 for the knife 35 is mounted on a fastening ring 42 which can be snapped in close-fitting manner onto a recess 43 of the free end 44 of outer tube 31 whereby exchanging of knife 35 is realized with a mounting 42.

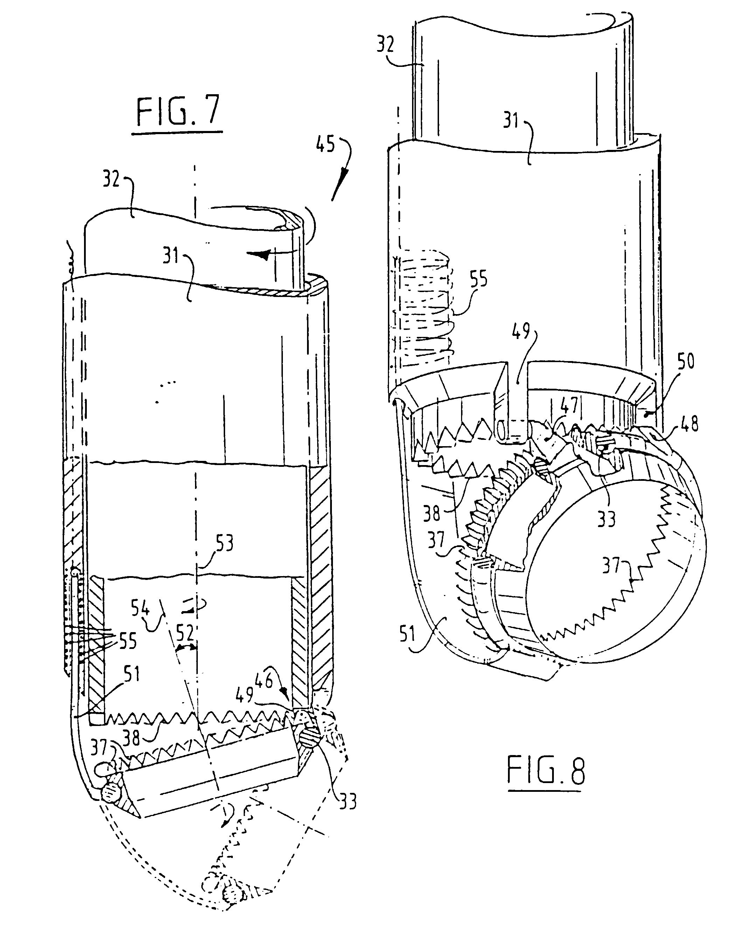

Figures 7 and 8 show another morcellator 45 according to the invention. In this case the bearing ring 33 is connected via hinge means 46 to outer tube 31. Hinge means 46 comprise two pivoting legs 47 and 48 connected to ring 33 and disposed such that a hinge shaft 49 passes through the pivoting legs at the position where toothings 37 and 38 comb with one another. Pivoting legs 47 and 48 are connected via respective pivoting legs 49 and 50 to outer tube 31.

The ring 33 is connected on the other side to outer tube 31 via a hinge member 51 in order to adjust the angle between the morcellator axis 53 and the knife axis. The hinge member is embodied in this case in memory metal and can assume different pivot positions depending on heating using heating means 55 incorporated in outer tube 31. It will however be apparent that other types of hinge members are possible, for instance mechanical hinge members which can be operated from outside the abdomen.

It is further noted that toothings 37 and 38 are embodied such that an adequate drive of knife 35 is possible irrespective of the adjusted angle 52.

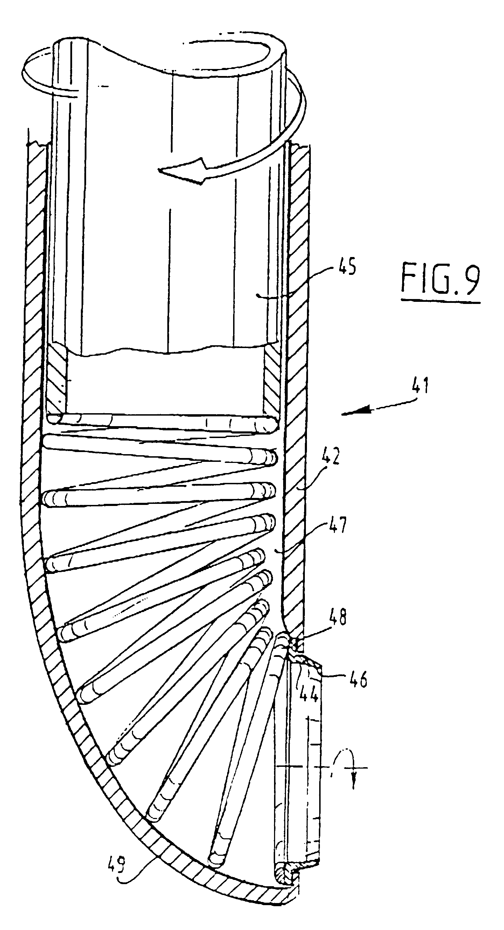

Figure 9 shows a morcellator 41 according to the invention which is provided with an outer tube 42 with a curved end 43 such that an opening 44 is directed laterally. Received rotatably in outer tube 42 is an inner tube 45 which is connected to the annular cutting means 46 via a spiral spring 47. By means of the spiral spring 47 the rotation of inner tube 45 is transmitted to the cutting means 46 which are mounted and pressed in a bearing 48 for that purpose.

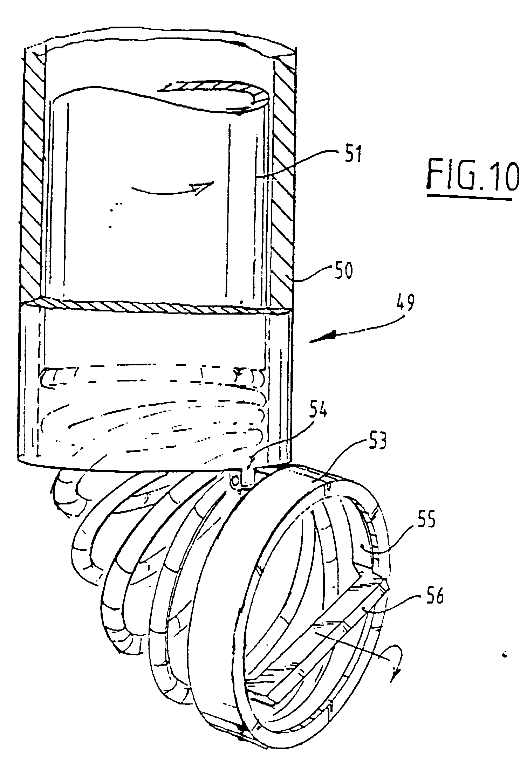

Figure 10 shows a morcellator 49 comprising an outer tube 50 and an inner tube 51 rotatable herein. The inner tube is connected with interposing of a spiral spring 52 to a ring 53 which is connected via hinge means 54 to outer tube 50. Mounted in ring 53 is an inner ring 55 which bears a cutting knife 56. Longitudinal downward displacement of inner tube 51 results in outward folding of the ring 53 while the knife 53 still rotates therein. It is thus possible to perform a lateral cutting movement with morcellator 49.

It is finally noted in respect of figures 9 and 10 that optionally the whole inner tube 45 respectively 51 can be embodied as a relatively stiff but rotatable spiral spring.

The position of the knife is such that it is possible to cut in horizontal direction (laterally), the knife angle then amounting to for instance about 45°.

## Claims

1. Tissue morcellator comprising a tube provided at one end with rotatable cutting means which are left at least partly free radially relative to the tube.

2. Morcellator as claimed in claim 1, wherein the cutting means lie at an angle of 10-90°, preferably 20-80°, relative to the tube axis.

3. Morcellator as claimed in claim 2, wherein the angle is adjustable relative to the tube axis.

4. Morcellator as claimed in claims 1-3, wherein the cutting means and the drive means connected thereto are mounted in an inner tube received in the morcellator tube.

5. Morcellator as claimed in claim 4, wherein the inner tube is substantially clear.

6. Morcellator as claimed in claim 4 or 5, wherein the inner tube is embodied as or is connected to the cutting means via a spiral spring.

7. Morcellator as claimed in claims 4-6, wherein discharge means are arranged in the inner tube for discharging severed tissue.

8. Morcellator as claimed in claims 1-7, wherein the cutting means are mounted with bearing means on an outer tube and drivable with an inner tube received in the outer tube.

9. Morcellator as claimed in claim 8, wherein the bearing means of the cutting means are connected to a fastening ring mounted releasably on the outer tube.

10. Morcellator as claimed in claim 8 or 9, wherein the bearing means are connected via hinge means and via a hinge member to the outer tube.

11. Morcellator as claimed in claim 10, wherein the hinge member is a member provided with heating means and containing memory metal.
